# EUROPÄISCHE PATENTSCHRIFT

(11) **EP 1 566 382 B1**
(45) Veröffentlichungstag und Bekanntmachung des Hinweises auf die Patenterteilung: **23.05.2007**
(21) Anmeldenummer: 04000931.8
(22) Anmeldetag: 17.01.2004
(51) Int. Cl.: C07D 251/62

(54) **Verfahren zur schonenden Abkühlung und Kristallisation von Melamin aus einer Melaminschmelze oder aus der Gasphase**
Process for the gentle cooling and crystallisation of melamin from a melamin melt or from the liquid phase
Procédé de refroidissement et crystallisation de mélamine a partir d'une fusion de mélamine ou de la phase gaseuse

(43) Veröffentlichungstag der Anmeldung: 24.08.2005
(73) Patentinhaber: Casale Chemicals S.A., 6900 Lugano-Besso (CH)
(72) Erfinder: Casale Chemicals S.A., 6900 Lugano-Besso (CH)
(74) Vertreter: Zardi, Marco

(56) Entgegenhaltungen:
- EP-A- 1 035 117
- US-A- 5 514 796

## Beschreibung

Melamin wird technisch nach zwei Verfahrensprinzipien durch Zersetzung von Harnstoff bei ca. 400 °C und in Gegenwart von Ammoniak hergestellt: entweder durch Umwandlung von Harnstoff in Gegenwart eines Katalysators bei niedrigen Drücken (bis 2 MPa) oder bei hohen Drucken (7 - 15 MPa) in einer rein thermischen Umsetzung. Die verschiedenen Verfahren sind in " Ullmann's Encyclopedia of Industrial Chemistry, Vol. A 16, 5th. Edit., Seiten 171 - 185, (**1990**) " beschrieben.

Bei den katalytischen Niederdruckverfahren verlässt das Melamin den Reaktor gasförmig, das Reaktionsgas enthält neben Melamin noch Ammoniak, Kohlendioxid und Zersetzungs produkte des nicht zu Melamin umgesetzten Harnstoffs. Beim BASF - Verfahren wird das Melamin - nach vorheriger Abtrennung von Katalysatorstaub und Melem - durch Zugabe von kaltem Reaktionsgas aus der Gasphase zu feinkörnigem Melamin desublimiert. Nachteil dieser Verfahrensweise ist, dass hohe Kühlgasmengen erforderlich sind, um das Melamin aus der Gasphase abzuscheiden und ein sehr feinkörniges Melamin anfällt. Da sich bei diesem Verfahren nach der Melamindesublimation kein weiterer Reinigungsschritt anschließt, werden hohe Anforderungen an die Reaktionsführung gestellt, um die Bildung von Nebenprodukten zu vermeiden.

Bei den katalytischen Verfahren der Chemie Linz und der DSM wird das Melamin aus der Gasphase durch Quenchen mit einer wässrigen Melaminsuspension abgeschieden. Nachteil des Wasserquenchens ist, das während des Quenchens ein Teil des Melamins zu Oxotriazinen hydrolysiert wird, wodurch eine Umkristallisations des Melamins notwendig wird sowie eine aufwendige Abwasseraufarbeitung erforderlich ist. Ein weiterer Nachteil besteht darin, dass nach der Abtrennung des Melamins ein wasserdampfhaltiges NH₃/CO₂- Gas anfällt, das nicht einer Harnstoffwäsche unterzogen werden kann.

Bei den nichtkatalytischen Hochdruckverfahren fällt das Melamin im Reaktor als Melaminschmelze bei ca. 400 °C und Drucken von 7 bis 15 MPa an. Die Schmelze enthält je nach den Verfahrensbedingungen noch mehr oder weniger hohe Anteile von Ammoniak und Kohlendioxid sowie durch Nebenreaktionen entstandene Verunreinigungen des Melamins wie Ureidomelamin, Melam, Melem etc. Das Problem besteht darin, dass Melamin bei 400 °C nur bei hohen Ammoniakdrucken stabil ist. Zur Abkühlung und Gewinnung des Melamins muss die Melaminschmelze jedoch aus dem Reaktor auf niedrigere Drucke entspannt und auf Temperaturen unterhalb 350 °C, besser auf 200 bis 300 °C abgekühlt werden. Das schnelle Abkühlen zur Vermeidung von NH₃-Abspaltungen aus dem Melamin ist technisch nur sehr aufwendig durchzuführen. Erfolgt dieses Abkühlen nicht schnell genug, entstehen durch Abspaltung von Ammoniak höhermolekulare Kondensationsprodukte wie Melam und Melem, die bei der späteren Weiterverarbeitung des Melamins stören Ein effektives Quenchen ist deshalb Vorbedingung für die Gewinnung von Melamin ausreichender Reinheit ( > 99,8 %).

Technisch kann die Abkühlung der Melaminschmelze und Umwandlung der Nebenprodukte wie Melam oder Ureidomelamin nach zwei Verfahrensprinzipien erfolgen:
a)Durch Quenchen mit einer wässrigen, alkalisierten Melaminsuspension oder - lösung ( Nissan ― Verfahren, US 3,637,686, DE 10229100A, WO 0029393) oder
b)Durch Quenchen mit flüssigen Ammoniak (US 5514796, US 02007061, WO 00/55142).

Das Quenchen mit einer wässrigen, alkalisierten Melaminsuspension oder -lösung hat zwangsläufig eine verstärkte Hydrolyse des Melamins zu Oxotriazinen zur Folge und damit einen Ausbeuteverlust von 2- 6 % (DE 100 30 453 A 1). Durch die Bildung der Hydrolyseprodukte muss kontinuierlich ein Teil der Mutterlauge ausgeschleust und aufgearbeitet werden, es entsteht eine Abwasserproblematik.

Die Verfahren zur " trockenen" Abscheidung mit flüssigem oder kaltem Ammoniak haben sich in der Praxis bisher nicht durchgesetzt. Die Gründe hierfür sind vielfältig: zum einen ist der Wärmübergang zwischen kaltem Gas und heißer Melaminschmelze nicht so optimal, dass die Bildung von Deammonsierungsprodukten während der Druckentspannung vermieden wird. Zum anderen stellen die bei der Abkühlung der Melaminschmelze entstehenden und aufzuarbeitenden großen Gasmengen einen wirtschaftlichen Nachteil dar. Außerdem ist das durch eine Ammoniak-Quench abgeschiedene Melamin sehr feinkörnig, seine mehlartige Konsistenz erschwert das weitere Handling. Die Nachteile der "trockenen" Abscheideverfahren sind beispielsweise im EP 1035117 ausführlich beschrieben.

Die vorliegende Erfindung vermeidet diese Nachteile. Sie betrifft ein Verfahren zur kontinuierlichen und schonenden Abkühlung und Kristallisation des Melamins aus einer Melaminschmelze oder aus der Gasphase ohne thermische Zersetzung oder Hydrolyse des Melamins während der Abkühlung und des Kristallisationsvorganges. Das Verfahren ist dadurch gekennzeichnet, dass eine Melaminschmelze, wie sie in technischen, nicht katalytischen Hochdruckverfahren zur Melaminherstellung anfällt, oder heißes Reaktionsgas aus einem katalytischen Prozess, das gasförmiges Melamin neben Ammoniak, Kohlendioxid und nicht umgesetzten Harnstoff enthält, direkt in eine flüssige, organische Phase entspannt oder eingeleitet wird. Als flüssige, organische Phase geeignet sind mehrwertige Alkohole wie Ehtylenglykol, Propylenglykol, Glycerin und/oder andere höherwertige Alkohole bzw. Mischungen dieser Alkohole sowie Ethanolamine der allgemeinen Formel RₙN(C₂H₅OH) ₃₋ₙ, wobei R einen aliphatischen Rest oder ein H- Atom bedeutet. Geeignet sind insbesondere Ethanolamine wie Mono-, Di- und Triethanolamin, Methyldiethanolamin bez. deren Mischungen. Die flüssige, organische Phase kann auch aus Mischungen der beiden genannten Verbindungsgruppen bestehen.

Die Temperatur der flüssigen, organischen Phase wird während des Abkühlungs- und Kristallisationsvorganges, d. h. während des sog. Quenchens, vorzugsweise zwischen 20°C und 350 °C gehalten, der Druck kann Atmosphärendruck betragen oder bis leicht unter dem Verfahrensdruck des jeweiligen Herstellungsverfahrens liegen. Des Weiteren kann die Menge der zum Quenchen benötigten flüssigen, organischen Phase so gewählt werden, dass sich das gesamte Melamin in der flüssigen Phase löst oder eine Suspension aus Melamin und flüssiger Phase bildet. Im ersteren Fall wird in einem nachfolgenden Verfahrensschritt das Melamin z.B. durch Abkühlung aus der flüssigen Phase auskristallisiert und abgetrennt, im letzteren Fall kann das Melamin ohne Abkühlung direkt durch Hydrocyclone und/oder Zentrifugen von der flüssigen Phase abgetrennt werden. Das Verfahren ist weiterhin dadurch gekennzeichnet, dass nach der Melaminabtrennung die flüssige Phase in den Quenchprozess zurückgeführt wird. Wegen der guten Wasserlöslichkeit der genannten flüssigen, organischen Phasen kann das Melamin nach der Abtrennung in den Zentrifugen mittels Wasserwäsche von allen Rückständen befreit werden.

Überraschend zeigte sich, dass durch Quenchen von gasförmigen Melamin oder einer Melaminschmelze mit den genannten organischen Verbindungen bzw. deren Mischungen eine so schnelle Abkühlung des gasförmigen Melamins bez. der Melaminschmelze erfolgt, dass keine Deammonisierungprodukte gebildet werden. Überraschenderweise entstanden auch bei hohen Quenchtemperaturen keine Hydrolyseprodukte durch Reaktion mit den OH-Gruppen der Alkohole wie bei einem Quench mit Wasser. Überraschend war ebenfall die hohe Löslichkeit des Melamins in den angeführten flüssigen, organischen Phasen, was einen großen verfahrenstechnischen Vorteil gegenüber einer Suspensionsfahrweise darstellt.

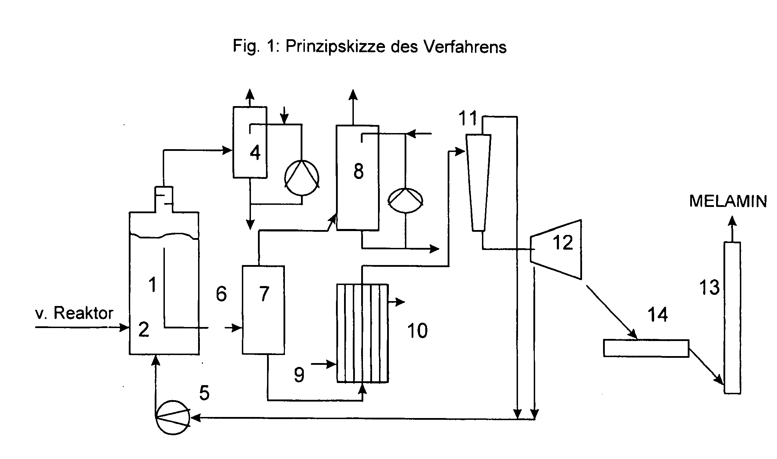

Im Folgenden wird das Verfahren anhand der Fig. 1 am Beispiel einer Melaminschmelze aus einem nichtkatalytischen Hochdruckverfahren näher beschrieben. Bei dem erfindungsgemäßen Verfahren kann jede aus einem technischen Hochdruckverfahren stammende Melaminschmelze eingesetzt werden. Entsprechendes gilt für gasförmiges Melamin aus einem katalytischen Verfahren. Die in der Beschreibung des Verfahrens angeführten Reaktionsbedingungen bedeuten keine Einschränkung, sie dienen nur zur Erläuterung und dem Verständnis des Verfahrens. Es ist dem Fachmann klar, dass die jeweiligen Betriebsbedingungen auf die physikalischen und chemischen Eigenschaften der eingesetzten flüssigen, organischen Phase abgestimmt werden müssen.

Die Eintragung der weitgehend von CO₂ und überschüssigen NH₃ befreite Melaminschmelze in den Quencher (1) erfolgt bevorzugt durch Eindüsen der Melaminschmelze über eine oder mehrere Düsen (2) in die vorgelegte flüssige, organische Phase, um eine möglichst schnelle Abkühlung zu erreichen. Es ist jedoch gleichfalls möglich, die Melaminschmelze am Kopf des Quenchers über ein Düse (Einstoff- oder Zweistoff-Düse) fein zu verteilen und durch weitere Sprühkegeldüsen die flüssige, organische Phase aufzugeben. Wichtig ist, dass der Kontakt zwischen Melaminschmelze und flüssiger, organischer Phase schnell und intensiv erfolgt.

Die Temperatur im Quencher (1) wird zwischen 20 °C und 350 °C gehalten, besonders vorteilhaft sind Temperaturen zwischen 100 °C und 250 °C , um einerseits eine genügende Löslichkeit des Melamins in der organischen Phase sicherzustellen, andererseits eine Zersetzungsreaktionen der flüssigen Phase zu vermeiden. Optimal sind Temperaturen, die - abhängig vom den Druckverhältnissen im Quencher (1) und Stripper (7) - unterhalb des Siedepunktes der eingesetzten flüssigen organischen Phase liegen.

Der Druck im Qencher richtet sich nach dem Verfahrensdruck des Prozesses. Hochdruckanlagen arbeiten im allgemeinen mit einem Reaktordruck von 7 bis 17 MPa, eine Druckdifferenz von 0,1 - 0,5 MPa zwischen Melaminreaktor und Quencher ist ausreichend, um eine genügend feine Versprühung des Melamins im Quencher zu erreichen. Bei den katalytischen Niederdruckverfahren ist der Druck im Quencher entsprechend niedriger zu wählen, außerdem empfiehlt sich in diesem Fall die Aufgabe des Reaktionsgases und der flüssigen Phase durch Düsen am Kopf des Quenchers, wie oben beschrieben.

Je nach den gewählten Druck- und Temperaturbedingungen im Quencher und der Menge der noch in der Melaminschmelze enthaltenen Gase ( NH₃, CO₂, HNCO ), entgast ein Teil dieser Komponenten, sammelt sich im Oberteil des mit Flüssigabscheider versehenen Quenchers und wird über das Druckhalteventil (3) in die Waschkolonne (4) entspannt und weiter aufgearbeitet, z.B. durch eine Wasserwäsche.

Die Umlaufmenge der in den Quencher über die Pumpe (5) eingetragenen flüssigen, organischen Phase, im Beispiel wird entweder so bemessen, dass die den Quencher verlassenden Lösung 5 - 8 Gew. % Melamin bei einer Austrittstemperatur von 100 °C enthält bzw. bei höheren Quenchtemperaturen entsprechend mehr (bis 40 Gew.%).

Alternativ können Temperatur und Umlaufmenge so abgestimmt werden, dass eine Melaminsuspension anfällt, aus der nach Abkühlung das suspendierte Melamin zusammen mit auskristallisiertem Melamin durch Hydrocyclone und/oder Zentrifugen abgetrennt wird. Die Verweilzeit der Melaminlösung oder der Melaminsuspension im Quencher kann so eingestellt werden, dass Deammonisierungsprodukte des Melamins wie Melam und Melem wieder in Melamin umgewandelt werden. Da gegenüber einem Wasserquench höhere Temperaturen im erfindungsgemäßen Verfahren angewandt werden können, erfolgt die Rückbildung dieser Komponenten zu Melamin wesentlich schneller. In Abhängigkeit vom Melam- und Melemgehalt der Melaminschmelze sind Verweilzeiten von 10 - 60 Minuten vorzugsweise 10 bis 30 Minuten ausreichend.

Aus dem Quencher (1) wird die melaminhaltige Lösung oder Suspension über das Entspannungsventil (6) in den NH₃-Stripper (7) entspannt. Bei einem Druck von 0,1 - 2 MPa im Stripper werden die restlichen gelösten Gase aus der Lösung oder Suspension ausgetrieben und in der Waschkolonne (8) weiter aufgearbeitet.

Die am Boden des NH₃-Strippers über das Ventil (9) abgezogene klare Lösung oder Suspension wird im Kühler (10) abgekühlt, wobei Melamin weitgehend auskristallisiert. Im Hydrocyclon (11) wird der größte Teil der abgeschiedenen Melaminkristalle abgetrennt und in der Zentrifuge (12) mit Kondensat rückstandsfrei gewaschen. Im Trockner (13) wird das Melamin getrocknet. Die im Waschwasser der Zentrifuge enthaltenen Reste an flüssiger, organischer Phase werden in einer separaten Kolonne zurück gewonnen.

Um den Anteil an organischer Phase im Waschwasser gering zu halten, kann das Melamin zunächst ohne Waschvorgang durch Zentrifugen abgetrennt und in einer nachgeschalteten separaten Apparatur, z.B. einem Bandfilter (14), durch Waschen von den anhaftenden Resten der organischen Phase befreit werden.

Der Überlauf der Hydrocyclone, der noch feinkörnige Melaminkristalle enthält, wird über die Pumpe (5) in den Quencher (1) zurückgeführt. Durch die Verwendung von Hydrocyclonen vor der eigentlichen Melaminabtrennung durch Zentrifugieren wird erreicht, dass das gewonnene Melamin eine gleichmäßige Korngrößenverteilung besitzt ohne größeren Feinstaubanteil.

Die nachfolgenden Beispiele demonstrieren die chemischen Grundlagen des erfindungsgemäßen Verfahrens am Beispiel von Ethylenglykol als flüssige, organische Phase, ohne hiermit die Eignung und Verwendung der anderen genannten Komponenten irgendwie einzuschränken.

### Beispiel 1

Ein ammoniakgesättigte Melaminschmelze mit einem Ausgangsgehalt von 9500 ppm Melam und 500 ppm Melem wurde mit einer Temperatur von 400 °C und einem Druck von 12 MPa aus einem Autoklaven in einen zweiten Autoklaven entspannt, der Ethylenglykol von 250 °C unter einem Druck von 7 MPa enthielt. Nach einer Verweilzeit von 30 Minuten unter diesen Bedingungen wurde die entstandene Lösung von Melamin in Ethylenglykol abgekühlt und das auskristallisierte Melamin sowie die Mutterlauge analysiert.

In der Mutterlauge konnten keine Zersetzungsprodukte des Ethylenglykols nachgewiesen werden.

Ebenso war das auskristallisierte Melamin frei von Oxotriazinen, der Melamgehalt war auf unter 1000 ppm abgesunken, der Melemgehalt war < 100 ppm.

### Beispiel 2

Ein melaminhaltiges Gas von 350 °C mit der Zusammensetzung 2 Vol% Melamin, 6 Vol.% Kohlendioxid und 92 Vol% Ammoniak wurde bei Atmophärendruck in 150 °C heißes Ehylenglykol eingeleitet. Aus der entstandenen Melamin/Ehylenglykol - Lösung mit einem Melamingehalt von 6,5 Gew. % wurde das Melamin durch Abkühlen auf 50 °C auskristallisiert. Nach dem Waschen hatte das Melamin eine Reinheit von 99,8 %, in der Mutterlauge konnten keine Oxotriazine oder Zersetzungsprodukte des Ethylenglykols nachgewiesen werden.

## Patentansprüche

1. Verfahren zur kontinuierlichen Abkühlung und Kristallisation von festem Melamin aus der Gasphase oder aus Melaminschmelzen wie sie bei den technischen Verfahren zur Herstellung von Melamin anfallen, **dadurch gekennzeichnet, dass** ein melaminhaltiges Prozessgas oder eine ammoniakgesättigte Melaminschmelze aus einem Hochdruckverfahren mit einer flüssigen, organischen Phase in Kontakt gebracht bzw. gequencht wird, dabei ohne die Bildung von Zersetzungs- oder Hydrolysprodukten abkühlt, in eine Lösung oder Suspension von Melamin in dieser flüssigen, organischen Phase überführt wird aus der durch weitere Abkühlung und Kristallisation das Melamin gewonnen und die flüssige organische Phase in den Prozess zurückgeführt wird.

2. Verfahren nach Anspruch 1 **dadurch gekennzeichnet, dass** die flüssige organische Phase aus mehrwertigen Alkoholen oder deren Mischungen besteht, insbesondere aus Ethylenglykol, Propylenglykol, Glycerin oder deren Mischungen.

3. Verfahren nach Anspruch 1 **dadurch gekennzeichnet, dass** die flüssige, organische Phase aus Ethanolaminen der allgemeinen Formel RₙN(C₂H₅OH)ₙ₋₃ besteht, worin R ein Wasserstoffatom oder einen aliphatischen Rest bedeutet, insbesondere Methyldiethanolamin, Mono-, Di- , oder Triethanolamin oder Mischungen dieser Verbindungen.

4. Verfahren nach mindestens einem der vorgenannten Ansprüche **dadurch gekennzeichnet, dass** das Abkühlen oder Quenchen melaminhaltiger Prozessgase oder einer Melaminschmelze mit einer flüssigen organischen Phase nach Anspruch 2 und 3 bei Temperaturen zwischen 100 °C und 350 °C, vorzugsweise bei Temperaturen von 100 °C bis 300 °C erfolgt.

5. Verfahren nach mindestens einem der vorgenannten Ansprüche **dadurch gekennzeichnet, dass** das Quenchen einer Melaminschmelze mit einer flüssigen organischen Phase bei einem Druck von 1 bis 13,5 MPA erfolgt, vorzugsweise bei 3 bis 8 MPa.

6. Verfahren nach mindestens einem der vorgenannten Ansprüche **dadurch gekennzeichnet, dass** die im Quencher anfallende Melaminlösung oder -suspension vor der Melaminabtrennung oder weiteren Kristallisation in einen Abscheider auf 0,1-0,5 MPa Druck entspannt und dabei die noch enthaltenen Gase wie Ammoniak, Kohlendioxid und Zersetzungsprodukte des nicht umgesetzten Harnstoffs aus der Lösung oder Suspension entfernt werden.

7. Verfahren nach mindestens einem der vorgenannten Ansprüche **dadurch gekennzeichnet, dass** die Ammoniakkonzentration in der flüssigen Phase des Quenchers nicht über 40 Gew.% ansteigt, vorzugsweise unter 20 Gew.% gehalten wird.

8. Verfahren nach mindestens einem der vorgenannten Ansprüche **dadurch gekennzeichnet, das** das in der flüssigen organischen Phase gelöste Melamin durch Kühlungskristallisation auskristallisiert wird, die gebildeten Melaminkristalle zusammen mit suspendierten Melamin durch Hydrocyclone und Zentrifugen abgetrennt, die Mutterlauge in den Prozess zurückgeführt und die abgetrennten Melaminkristalle in einer separaten Einheit mit Wasser gewaschen werden.

9. Verfahren nach mindestens einem der vorgenannten Ansprüche **dadurch gekennzeichnet, dass** die Verweilzeit des abgeschiedenen oder gelösten Melamins im Quencher je nach den gewählten Temperatur- und Druckbedingungen so abgestimmt wird, dass vorhandene Nebenprodukte wie Melem und Melam wieder zu Melamin umgesetzt werden.

## Claims

1. Process for the continuous cooling and crystallization of solid melamine from the gaseous phase or from melamine melts like those occurring in industrial processes for the production of melamine, **characterized in that** a melamine-containing process gas or an ammonia-saturated melamine melt from a high-pressure process is brought in contact or quenched with a liquid organic phase and thereby cooled without formation of products of decomposition or hydrolysis, and converted into a solution or suspension of melamine in this liquid organic phase from which the melamine is extracted through further cooling and crystallization, and the liquid organic phase is recirculated into the process.

2. Process according to Claim 1, **characterized in that** the liquid organic phase consists of polyvalent alcohols or mixtures thereof, and in particular ethylene glycol, propylene glycol, glycerine, or mixtures thereof.

3. Process according to Claim 1, **characterized in that** the liquid organic phase consists of ethanolamines of the general formula RₙN(C₂H₅OH)ₙ₋₃ where R stands for a hydrogen atom or an aliphatic group, and in particular methyldiethanolamine, mono-, di- or triethanolamine, or mixtures of these compounds.

4. Process according to at least one of the preceding claims, **characterized in that** the cooling or quenching of melamine-containing process gas or melamine melt with a liquid organic phase according to Claims 2 and 3 is conducted at temperatures between 100°C and 350°C, preferably at temperatures of 100°C to 300°C.

5. Process according to at least one of the preceding claims, **characterized in that** the quenching of a melamine melt with a liquid organic phase is conducted at a pressure of 1 to 13.5 MPa, preferably at 3 to 8 MPa.

6. Process according to at least one of the preceding claims, **characterized in that** the melamine solution or suspension formed in the quencher is decompressed to 0.1-0.5 MPa in a separator before melamine separation or further crystallization takes place, and the gases still contained such as ammonia, carbon dioxide and products of decomposition of unconverted urea are thereby removed from the solution or suspension.

7. Process according to at least one of the preceding claims, **characterized in that** the ammonia concentration in the liquid phase of the quencher does not rise above 40 wt% and is preferably kept below 20 wt%.

8. Process according to at least one of the preceding claims, **characterized in that** the melamine dissolved in the liquid organic phase is crystallized out by cooling crystallization, the melamine crystals formed, together with suspended melamine, are separated by hydraulic cyclones and centrifuges, the mother liquor is recirculated into the process, and the separated melamine crystals are washed with water in a separate unit.

9. Process according to at least one of the preceding claims, **characterized in that** the dwell time of the separated or dissolved melamine in the quencher is adjusted in accordance with the chosen temperature and pressure conditions so that by-products present such as melem and melam are reconverted into melamine.

## Revendications

1. Procédé de refroidissement et de cristallisation continus de mélamine solide à partir de la phase gazeuse ou de masses fondues de mélamine, telles qu'elles sont produites dans les procédés industriels de préparation de mélamine, **caractérisé en ce qu'**un gaz de fabrication d'un procédé sous haute pression contenant de la mélamine ou une masse fondue de mélamine saturée en ammoniac est amené au contact et/ou refroidi par injection d'une phase organique liquide, sans refroidir la réaction de formation de produits d'hydrolyse ou de décomposition, **en ce qu'**il est transféré dans une solution ou suspension de mélamine dans cette phase organique liquide, à partir de laquelle la mélamine est obtenue par refroidissement et cristallisation supplémentaires, et **en ce que** la phase liquide organique est recyclée dans le processus.

2. Procédé suivant la revendication 1, **caractérisé en ce que** la phase organique liquide se compose d'alcools polyvalents ou de mélanges de ces derniers, en particulier d'éthylèneglycol, propylèneglycol, glycérine, ou de mélanges de ces derniers.

3. Procédé suivant la revendications 1, **caractérisé en ce que** le phase organique liquide se compose d'éthanolamines de formule générale RₙN(C₂H₅OH)ₙ₋₃, dans laquelle R désigne un atome d'hydrogène ou une chaîne aliphatique, en particulier méthyldiéthanolamine, mono-, di-, ou triéthanolamine ou des mélanges de ces composés.

4. Procédé suivant l'une au moins des revendications précédentes, **caractérisé en ce que** le refroidissement ou refroidissement par injection de gaz de fabrication contenant de la mélamine ou une masse fondue de mélamine s'effectue avec une phase organique liquide suivant les revendications 2 et 3, à des températures comprises entre 100°C et 350°C, de préférence à des températures comprises entre 100°C et 300°C.

5. Procédé suivant l'une au moins des revendications précédentes, **caractérisé en ce que** le refroidissement par injection d'une fusion de mélamine s'effectue avec une phase organique liquide sous une pression de 1 à 13,5 MPa, de préférence de 3 à 8 MPa.

6. Procédé suivant l'une au moins des revendications précédentes, **caractérisé en ce que** la solution ou suspension de mélamine, produite dans le refroidisseur par injection, avant la séparation de la mélamine ou cristallisation ultérieure dans un séparateur, est détendue à une pression 0,1 - 0,5 MPa et les gaz encore contenus, tels que l'ammoniac, le gaz carbonique et les produits de décomposition de l'urée non convertie sont éliminés de la solution ou de la suspension.

7. Procédé suivant l'une au moins des revendications précédentes, **caractérisé en ce que** la concentration d'ammoniac dans la phase liquide du refroidisseur par injection ne s'élève pas à plus de 40% en poids et est, de préférence, maintenue au-dessous de 20% en poids.

8. Procédé suivant l'une au moins des revendications précédentes, **caractérisé en ce que** la mélamine, dissoute dans la phase organique liquide, est cristallisée par refroidissement, les cristaux de mélamine formés sont séparés avec la mélamine en suspension par hydrocyclones et centrifugeuses, la solution-mère est recyclée dans le processus et les cristaux de mélamine séparés sont lavés à l'eau dans une unité séparée.

9. Procédé suivant l'une au moins des revendications précédentes, **caractérisé en ce que** la durée de séjour de la mélamine séparée ou dissoute dans le refroidisseur par injection est adaptée, suivant les conditions de température et de pression choisies, de sorte que des produits secondaires présents, tels que melem et melam, sont reconvertis en mélamine.
